(19) Europäisches Patentamt
European Patent Office
Office européen des brevets

(11) EP 0 827 948 B1

(12) EUROPÄISCHE PATENTSCHRIFT

(45) Veröffentlichungstag und Bekanntmachung des Hinweises auf die Patenterteilung:
**14.11.2001 Patentblatt 2001/46**

(51) Int Cl.[7]: **C07C 69/96**, C08G 63/64

(21) Anmeldenummer: **97114808.5**

(22) Anmeldetag: **27.08.1997**

# (54) Gemische cyclischer Oligocarbonate, ihre Herstellung und Verwendung

Mixtures of cyclic oligocarbonates, process for their preparation and use thereof

Mélanges d' oligocarbonates cycliques, leur procédé de préparation et leur utilisation

(84) Benannte Vertragsstaaten:
**BE DE ES FR GB IT NL**

(30) Priorität: **09.09.1996 DE 19636539**

(43) Veröffentlichungstag der Anmeldung:
**11.03.1998 Patentblatt 1998/11**

(73) Patentinhaber: **BAYER AG**
**51368 Leverkusen (DE)**

(72) Erfinder:
- **Köhler, Burkhard, Dr.**
  **51373 Leverkusen (DE)**
- **Priddy Jr, Duane, Dr.**
  **47800 Krefeld (DE)**
- **Chen, Yun, Dr.**
  **47800 Krefeld (DE)**
- **Pielartzik, Harald, Dr.**
  **47800 Krefeld (DE)**
- **Kumpf, Robert, Dr.**
  **40489 Düsseldorf (DE)**

(56) Entgegenhaltungen:
**EP-A- 0 359 953** **DE-A- 4 029 808**
**US-A- 4 982 014**

- **Macromolecules 1991, vol. 24, 3035-3044**
- **Indian Journal of Technology, 1993, vol. 31, p. 234-246**

EP 0 827 948 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Gemäß EP 0 359 953 (Le A 26 344) sind die Diphenole der Formel (I) bekannt,

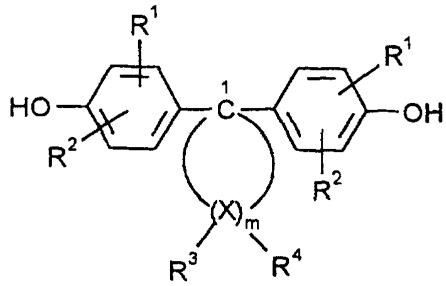

(I),

worin

$R^1$ und $R^2$ unabhängig voneinander Wasserstoff, Halogen, bevorzugt Chlor oder Brom, $C_1$-$C_8$-Alkyl, $C_5$-$C_6$-Cycloalklyl, $C_6$-$C_{10}$-Aryl, bevorzugt Phenyl, und $C_7$-$C_{12}$-Aralkyl, bevozugt Phenyl-$C_1$-$C_4$-Alkyl, insbesondere Benzyl,

m eine ganze Zahl von 4 bis 7, bevorzugt 4 oder 5,

$R^3$ und $R^4$ für jedes X individuell, unabhängig voneinander Wasserstoff oder $C_1$-$C_6$-Alkyl und

X Kohlenstoff bedeuten,

mit der Maßgabe, daß an mindestens einem Atom X, $R^3$ und $R^4$ gleichzeitig Alkyl bedeuten.

Bevorzugt sind an 1 bis 2 Atomen X, insbesondere nur an einem Atom X, $R^3$ und $R^4$ gleichzeitig Alkyl.

Bevorzugter Alkylrest ist Methyl; die X-Atome in α-Stellung zu dem di-phenyl-substituierten C-Atom (C-1) sind bevorzugt nicht dialkylsubstituiert, dagegen ist die Alkyl-disubstitution in β-Stellung zu C-1 bevorzugt. Besonders bevorzugt ist, daß ein X-Atom, in β-Stellung dialkylsubstituiert, und ein X-Atom in β'-Stellung monoalkylsubstituiert ist.

Bekannt sind darunter auch Dihydroxydiphenylcycloalkane mit 5 und 6 Ring-C-Atomen im cycloaliphatischen Rest (m = 4 oder 5 in Formel (I)) wie beispielsweise die Diphenole der Formeln

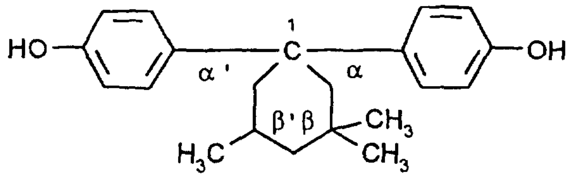

(II)

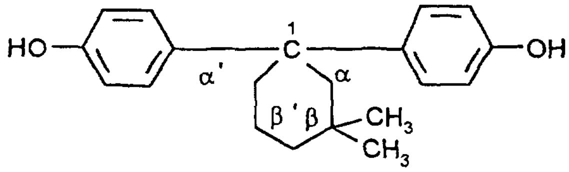

(III)

und

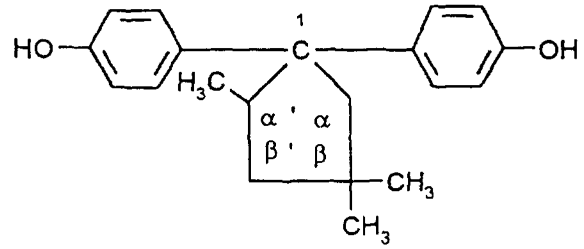 (IV),

wobei das 1,1-Bis(4-hydroxyphenyl)-3,3,5-trimethylcyclohexan (Formel II) technisch besonders bevorzugt ist.

In EP 0 359 953 sind auch die Mono- und Bischlorkohlensäureester der Diphenole der Formel (I) erwähnt (Seite 10, Zeilen 12-14 der EP; siehe auch US-PS 4 982 014, Spalte 9, Zeilen 6 bis 9).

Einzelheiten der Herstellung der Bischlorkohlensäureester sind nicht erwähnt, ebensowenig Gemische mit Chlorkohlensäureester-haltigen Vorphosgenaten.

Es hat sich nun gezeigt, daß man bei einer speziellen Herstellung der Bischlorkohlensäureester der Diphenole der Formel (I) diese im Gemisch mit den Chlorkohlensäureester-haltigen Vorphosgenaten enthält. Die Herstellung kann analog Makromolecules 1991, 24, S. 3035 - 3044 erfolgen.

Gegenstand der vorliegenden Erfindung sind somit Gemische aus Bischlorkohlensäureestern der Diphenole der Formel (I) mit Bischlorkohlensäureesterhaltigen Vorphosgenaten aus den Diphenolen der Formel (I), also Bischlorkohlensäureestergemische der Formel (V)

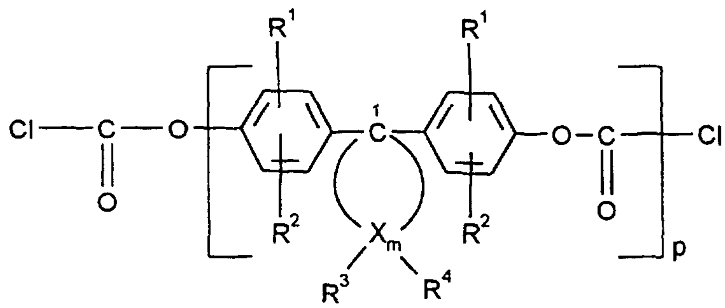 (V),

worin $R^1$ bis $R^4$, "m" und "X" die für Formel (I) genannte Bedeutung haben und "p", im Mittel eine Zahl von 1 bis 4 ist.

Gegenstand der vorliegenden Erfindung ist auch ein Verfahren zur Herstellung der Bischlorkohlensäureestergemische der Formel (V), das dadurch gekennzeichnet ist, daß man in ein zweiphasiges Gemisch aus einem organischen Lösungsmittel, vorzugsweise Methylenchlorid, mit Diphenolen der Formel (I) und Wasser Phosgen bei Temperaturen von -5°C bis +40°C einleitet und gleichzeitig die Lösung oder die Suspension von einem Alkalimetall- oder Erdalkalimetallhydroxid so zugibt, daß der pH-Wert zwischen 1 und 8, vorzugsweise 2 und 5 liegt.

Die erfindungsgemäßen Bischlorkohlensäureestergemische der Formel (V) sind insbesondere geeignet zur Herstellung von Gemischen von cyclischen, aromatischen Oligocarbonaten.

Gegenstand der Erfindung ist somit auch die Verwendung der Gemische der Formel (V) zur Herstellung von Gemischen von cyclischen, aromatischen Oligocarbonaten.

Die Umsetzung der Gemische der Formel (V) zu den Gemischen von cyclischen Oligocarbonaten erfolgt in der Weise, daß man die Lösung von (V) in einem organischen Lösungsmittel, vorzugsweise Methylenchlorid und ein organisches Amin, vorzugsweise Triethylamin, synchron zu einem zweiphasigen Gemisch aus einem organischen Lösungsmittel, vorzugsweise Methylenchlorid, und Wasser bei Temperaturen von 0°C bis 40°C, vorzugsweise 30°C bis 40°C zutropft, wobei gleichzeitig die Lösung oder Suspension von einem Alkalimetall- oder Erdalkalimetallhydroxid so zugegeben wird, daß der pH-Wert zwischen 7 und 13, vorzugsweise 9 und 11 liegt.

Die Herstellung kann analog Indian Journal of Technology 31 (1993) Seiten 234 - 246 erfolgen.

Die Gemische der cyclischen Oligocarbonate haben verschiedene Polykondensationsgrade "q" die im Mittel zwischen 2 und 20 liegen.

Gegenstand der vorliegenden Erfindung ist somit auch ein Verfahren zur Herstellung von Gemischen cyclischer Oligocarbonate der Formel (VI)

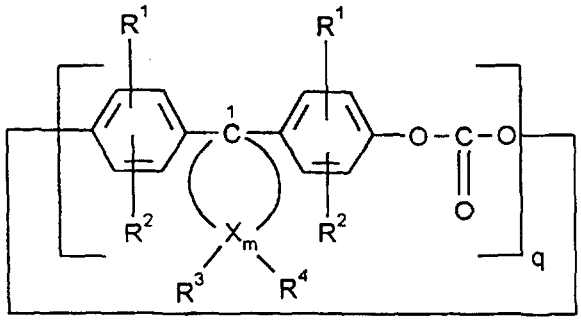

(VI),

worin $R^1$ bis $R^4$, "m" und "X" die für Formel (I) genannte Bedeutung haben und "q" im Mittel eine Zahl von 2 bis 20 ist, das dadurch gekennzeichnet ist, daß man man die Lösung von (V) in einem organischen Lösungsmittel, vorzugsweise Methylenchlorid und ein organisches Amin, vorzugsweise Triethylamin, synchron zu einem zweiphasigen Gemisch aus einem organischen Lösungsmittel, vorzugsweise Methylenchlorid, und Wasser bei Temperaturen von 0°C bis 40°C, vorzugsweise 30°C bis 40°C zutropft, wobei gleichzeitig die Lösung oder Suspension von einem Alkalimetall- oder Erdalkalimetallhydroxid so zugegeben wird, daß der pH-Wert zwischen 7 und 13, vorzugsweise 9 und 11 liegt.

Gegenstand der vorliegenden Erfindung sind außerdem die nach dem erfindungsgemäßen Verfahren erhältlichen Gemische cyclischer Oligocarbonate der Formel (VI).

Als Beweis, daß cyclische Strukturen vorliegen, dient die Ermittlung der Endgruppen, indem die phenolischen OH-Endgruppen photometrisch nach Anfärbung mit $TiCl_4$, und die Stickstoff-haltigen Endgruppen durch Verbrennung/Chemilumineszenz bestimmt werden.

Die erfindungsgemäß erhältlichen Oligocarbonatgemische haben Endgruppengehalte von maximal 0,3 Mol, vorzugsweise von maximal 0,1 Mol pro Mol Oligocarbonatgemisch.

Cyclische Oligocarbonate und ihre Herstellung sind bekannt. (Siehe beispielsweise DE-AS 1 229 101, US-PS 3 386 154, US-PS 4 727 134, J. Am. Chem. Soc. 1990, 112, Seiten 2399 - 2402, Macromolecules 1991, 24, Seiten 3035 - 3044 und Indian Journal of Technology, Vol. 31, 1993, Seiten 234-246). In diesen Literaturstellen sind jedoch nicht die Diphenole (I) einbezogen.

Andererseits können bei der Herstellung von Polycarbonaten aus den Diphenolen (I) gemäß EP-0 359 953 beziehungsweise US-PS 4 982 014 in untergeordneten Mengen cyclische Oligocarbonate mitentstehen. Die gemäß EP beziehungsweise US-PS erhältlichen Polycarbonatgemische haben aber Endgruppengehalte von mindestens 1,5 Mol, pro Mol Polycarbonatgemisch.

Gemäß US-PS 4 616 077 sind cyclische Oligocarbonate bekannt, die jedoch andere Struktureinheiten enthalten (siehe insbesondere US-PS 4 616 077, Spalte 2, Zeilen 52 ff - Spalte 3, Zeile 10).

Die nach den erfindungsgemäßen Verfahren erhältlichen Gemische der cyclischen Oligocarbonate der Formel (VI) lassen sich in bekannter Weise (siehe beispielsweise gemäß Indian Journal of Technology 31 (1993) Seiten 234-246) zu den hochmolekularen Polycarbonaten der EP 0 359 953 beziehungsweise des US-PS 4 982 014 durch ringöffnende Polymerisation umwandeln.

Gegenstand der vorliegenden Erfindung ist somit auch die Verwendung der Gemische der cyclischen Oligocarbonate der Formel (VI) zur Herstellung von hochmolekularen Polycarbonaten mit $\overline{M}_w$ von mindestens 10 000, vorzugsweise zwischen 10 000 und 500 000 ($\overline{M}_w$, Gewichtsmittelmolekulargewicht, ermittelt durch Gelpermeationschromatographie).

Gegenstand der vorliegenden Erfindung ist außerdem ein Verfahren zur Herstellung von hochmolekularen, thermoplastischen, aromatischen Polycarbonate mit $\overline{M}_w$ von mindestens 10 000, vorzugsweise zwischen 10 000 und 500 000 die an bifunktionellen Struktureinheiten nur solche der Formel (VII)

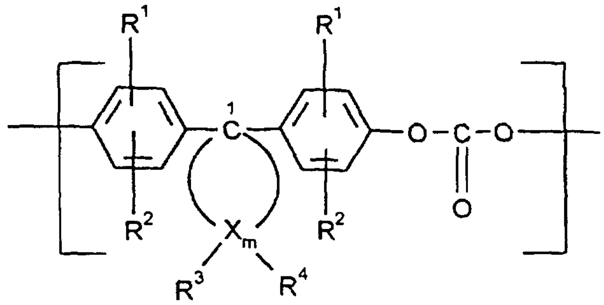

(VII),

haben, worin $R^1$ bis $R^4$, "m" und "X" die für Formel (I) genannte Bedeutung haben, das dadurch gekennzeichnet ist, daß man die cyclischen Oligocarbonatgemische der Formel (VI) in Gegenwart von 0,001 Gew.-% bis 0,1 Gew.-%, vorzugsweise 0,005 bis 0,05 Gew.-%, bezogen auf Oligocarbonatgemisch der Formel (VI), eines Katalysators 15 Sekunden bis 60 Minuten, vorzugsweise 3 Minuten bis 30 Minuten auf 200°C bis 400°C, vorzugsweise 220°C bis 350°C erhitzt.

Geeignete Katalysatoren für eine derartige anionische ringöffnende Polymerisation sind z. B. in US-P 4,650,852 und US-P 4,605,731 beschrieben. Bevorzugte Katalysatoren sind z. B. Tetrabutylammoniumtetraphenylboranat, Tetramethylammoniumtetraphenylboranat, Lithiumtetraphenylboranat, Tetramethylphosphoniumtetraphenylboranat, Tetrabutylphosphoniumtetraphenylboranat und Tetraphenylphosphoniumtetraphenylboranat.

Die Zahl und Art der Endgruppen in einem solchen hochmolekularen Polycarbonat ist durch unübersichtliche Nebenreaktionen oft nicht mehr nachvollziehbar.

Ein Vorteil dieses Verfahrens ist, daß man Formteile aus solch hochmolekularen und in der Schmelze hochviskosen Polycarbonaten erhalten kann, die normalerweise in der Schmelze nicht mehr verarbeitbar sind.

Die nach dem erfindungsgemäßen Verfahren erhältlichen Gemische der cyclischen Oligocarbonate der Formel (VI) lassen sich außerdem in Gegenwart von linearen, aromatischen Polycarbonaten aus anderen Diphenolen der Formel (VIII)

$$HO\text{-}Z\text{-}OH \qquad (VIII),$$

ringöffnend polymerisieren, oder in die linearen Polycarbonate aus Diphenolen der Formel (VII) ringöffnend einbauen, wobei Gemische von linearen Polycarbonaten aus solchen mit Struktureinheiten (VII) und solchen mit Struktureinheiten (IX)

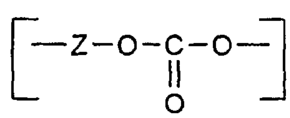

(IX),

und lineare Copolycarbonate mit Struktureinheiten (VII) und (IX) entstehen.

Die als Reaktionspartner einzusetzenden linearen Polycarbonate aus den anderen Diphenolen (VIII) haben mittlere Molekulargewichte $\overline{M}_w$ (Gewichtsmittel, ermittelt durch Gelpermeationschromatographie) von 8 000 bis 100 000, vorzugsweise von 10 000 bis 40 000 und Einfriertemperaturen (ermittelt gemäß Differentialthermoanalyse) von 140°C bis 180°C.

Daraus ergeben sich für die zweiwertigen aromatischen Reste -Z-, die 6 bis 30 C-Atome haben und gegebenenfalls noch -O-, -S- oder

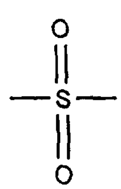

Einheiten und/oder Cl- oder Br-Substituenten enthalten können, und damit für die auszuwählenden Diphenole (VIII) gewisse Beschränkungen, die dem Fachmann geläufig sind.

Bevorzugte Diphenole für die als Reaktionspartner einzusetzenden linearen Polycarbonate sind solche der Formel (X)

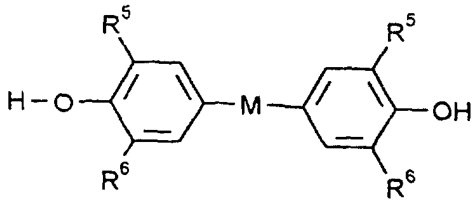 (X),

worin $R^5$ und $R^6$ gleich oder verschieden und H, Cl, Br oder $CH_3$ sind und -Meine Einfachbindung, -O-, -S-, $-SO_2-$, ein $C_1$-$C_5$-Alkyliden-Rest, ein $C_2$-$C_6$-Alkylen-Rest, der Benzyliden-Rest

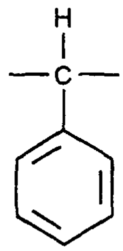 ,

der Phenethyliden-Rest

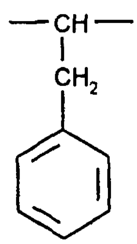

oder der Iso-phenethyliden-Rest

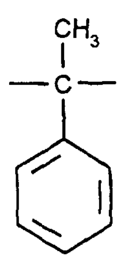

ist.

Um Molekulargewichte $\overline{M}_w$ und Einfriertemperaturen der linearen Polycarbonat-Reaktionspartner abzustimmen, können die linearen Polycarbonate auch aus Gemischen der anderen Diphenole (VIII) aufgebaut sein.

Das Gew.-Verhältnis der cyclischen Oligocarbonatgemische (VI) und der linearen, aromatischen Polycarbonat-Reaktionspartner aus den anderen Diphenolen (VIII) liegt zwischen 0,1 Gew.-% zu 99,9 Gew.-% und 80 Gew.-% zu 20 Gew.-%, vorzugsweise zwischen 10 zu 90 Gew.-% und 65 zu 35 Gew.-%.

Dieses Gew.-Verhältnis der Reaktionspartner ergibt dann jeweils ein entsprechendes Verhältnis der Struktureinheiten (VII) zu (IX) in den jeweils resultierenden Gemischen von linearen Polycarbonaten und resultierenden Copolycarbonaten.

Gegenstand der vorliegenden Erfindung ist somit auch die Verwendung der Gemische der cyclischen Oligocarbonate der Formel (VI) zur Modifizierung von linearen, aromatischen Polycarbonaten aus anderen Diphenolen der Formel (VIII) mit $\overline{M}_w$ (Gewichtsmittelmolekulargewicht, ermittelt durch Gelpermeationschromatographie) von 8 000 bis 100 000 und Einfriertemperaturen (ermittelt gemäß Differentialthermoanalyse) von 140°C bis 180°C, vorzugsweise von 145°C bis 152°C.

Die resultierenden Gemische von linearen Polycarbonaten sind beispielsweise aus der DE-OS 3 833 953 (Le A 26 397) bekannt, die resultierenden Copolycarbonate aus der bereits angezogenen EP 0 359 953 und der bereits angezogenden US-PS 4 982 014.

Im einzelnen entscheiden die Verfahrensbedingungen darüber, ob überwiegend Gemische von linearen Polycarbonaten oder ob Copolycarbonate entstehen.

Gegenstand der vorliegenden Erfindung ist somit auch ein Verfahren zur Herstellung von Gemischen von linearen Polycarbonaten aus solchen mit Struktureinheiten (VII) und solchen mit Struktureinheiten (IX) und linearen Copolycarbonaten mit Struktureinheiten (VII) und (IX), das dadurch gekennzeichnet ist, daß man

A) lienare, aromatische Polycarbonate

a) mit Struktureinheiten der Formel (IX)

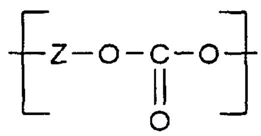

(IX),

worin -Z- ein zweiwertiger, aromatischer Rest mit 6 bis 30 C-Atomen ist, der gegebenenfalls noch -O-, -S- oder

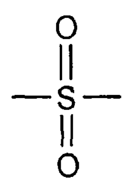

Einheiten und/oder Cl- oder Br-Substituenten enthalten kann,

b) mit Gewichtsmittelmolekulargewichten $\overline{M}_w$ von 8 000 bis 100 000, vorzugsweise von 10 000 bis 40 000 und

c) mit Einfriertemperaturen von 140°C bis 180°C, vorzugsweise von 145°C bis 152°C, und

B) Gemische cyclischer Oligocarbonate der Formel (VI)

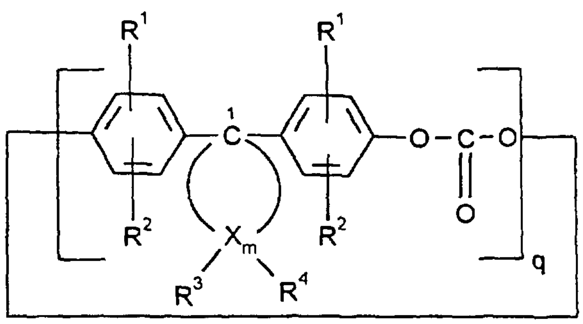

(VI),

worin $R^1$ bis $R^4$, "m" und "X" die für Formel (I) genannte Bedeutung haben und "q" im Mittel eine Zahl von 2 bis 20 ist,

in Gew.-Verhältnissen zwischen 0,1 Gew.-% Komponente B) zu 99,9 Gew.-% Komponente A) und 80 Gew.-% Komponente B) zu 20 Gew.-% Komponente A), bei Temperaturen von 200°C bis 400°C, vorzugsweise von 220 bis 350°C,

gegebenenfalls in Gegenwart von Katalysatoren, zwischen 10 Sekunden und 60 Minuten, vorzugsweise zwischen 30 Sekunden und 3 Minuten in der Schmelze vermischt.

Die erhaltenen Gemische der linearen Polycarbonate haben mittlere Gewichtsmittelmolekulargewichte $\overline{M}_w$ (ermittelt durch Gelpermerationschromatographie) von 8 000 bis 100 000, vorzugsweise von 10 000 bis 40 000. Sie haben außerdem eine verbesserte Einfriertemperatur (Glastemperatur) gegenüber den rein physikalischen Mischungen aus den Komponenten A) + B).
Ein Vorteil dieses Verfahrens ist, daß man nur eine Variante an linearen Polycarbonaten herstellen muß und eine anschließende Variation der Glastemperatur durch einen Mischungsschritt durchführen kann.
Die optionalen Katalysatoren sind Dibutylzinnoxid, Cobalt(II)acetattetrahydrat, Antimon(III)oxid, Mangan(II) acetattetrahydrat, Titan(IV)butoxid, Zinkacetat-dihydrat, Dibutylzinndilaurat, Zinn(II)acetattetramethyldiacetoxystannoxan, Zinn(IV)-oxid, Blei(II)acetattrihydrat, Dibutylzinndiacetat, Natriumbenzoat, und Titan(IV)-bis(ethylacetoacetat).
Beispiele für Bischlorkohlensäureestergemische der Formel (V) sind solche der Formel (Va)

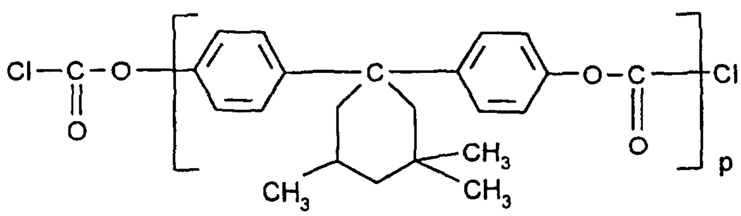

(Va),

wobei "p" den Wert von etwa1 1~1.3 hat, und (Vb)

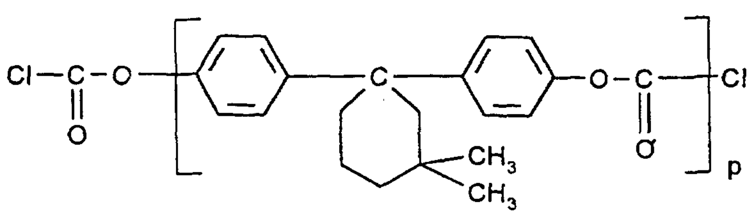

(Vb),

wobei "p" den Wert von etwa 1~1.3 hat.
Beispiele für Gemische cyclischen Oligocarbonate der Formel (VI) sind solche der Formeln (VIa)

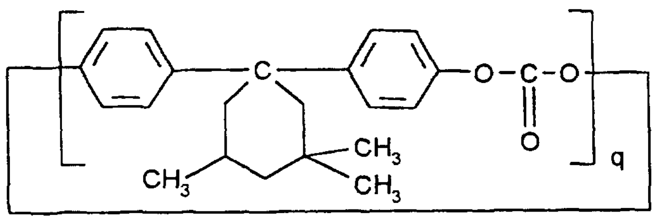

(VIa),

wobei "q" im Mittel 3 bis 4 ist.
Die Gemische cyclischer Oligocarbonate der Formel (VIa) enthalten 0,03 Mol Endgruppen pro Mol Gemische.
Beispiele für geeignete Diphenole (VIII) sind 4,4'-Dihydroxydiphenyl, 2,2-Bis-(4-hydroxyphenyl)-propan,

2,4-Bis(4-hydroxyphenyl)-2-methylbutan, 1,1-Bis-(4-hydroxyphenyl)-cyclohexan, α, α-Bis-(4-hydroxyphenyl)-p-diisopropylbenzol, 2,2-Bis-(3-methyl-4-hydroxyphenyl)-propan, 2,2-Bis-(3-chlor-4-hydroxyphenyl)-propan, Bis-(3,5-dimethyl-4-hydroxyphenyl)-methan, 2,2-Bis-(3,5-dimethyl-4-hydroxyphenyl)-propan, Bis-(3,5-dimethyl-4-hydroxyphenyl)-sulfon, 2,4-Bis-(3,5-dimethyl-4-hydroxyphenyl)-2-methylbutan, 1,1-Bis-(3,5-dimethyl-4-hydroxyphenyl)-cyclohexan, α, α-Bis-(3,5-dimethyl-4-hydroxyphenyl)-p-diisopropylbenzol, 2,2-Bis-(3,5-dichlor-4-hydroxyphenyl)-propan und 2,2-Bis-(3,5-dibrom-4-hydroxyphenyl)-propan und 2,2-Bis-(3-allyl-4-hydroxyphenyl)-propan.

Beispiele für bevorzugte Diphenole (VIIII) sind 2,2-Bis-(4-hydroxyphenyl)-propan, 2,2-Bis-(3,5-dimethyl-4-hydroxyphenyl)-propan, 2,2-Bis-(3,5-dichlor-4-hydroxyphenyl)-propan, 2,2-Bis-(3,5-dibrom-4-hydroxyphenyl)-propan, 1,1-Bis-(4-hydroxyphenyl)-cyclohexan und 2,2-Bis-(3-allyl-4-hydroxyphenyl)-propan.

Bei den beiden erfindungsgemäßen Verfahren zur Weiterverarbeitung der erfindungsgemäßen Gemische der cyclischen Oligocarbonate der Formel (VI) können während der Durchführung des Verfahrens die für Polycarbonate üblichen Additive, wie Entformungsmittel, Stabilisatoren gegen Hitze und UV-Licht, Flammschutzmittel etc. in den bekannten Mengen zugesetzt werden. (Nähere Einzelheiten siehe beispielsweise EP 0 359 953, Seite 12, Zeilen 20 ff und DE-OS 38 33 953, Seite 13, Zeile 54 ff).

Beiden erfindungsgemäßen Verfahren zur Weiterverarbeitung der Oligocarbonatgemische kann sich die Herstellung von beliebigen Formteilen direkt anschließen. Dies geschieht wie folgt: Bei der ringöffnenden Polymerisation der Oligocarbonatgemische erfolgt die Formgebung direkt bei der Polymerisation (reaction molding). Die Polycarbonat-Gemische erhalten aus cyclischen Oligocarbonaten und linearen Polycarbonaten werden wie üblich anschließend durch Spritzguß oder Extrusion geformt.

Die erhaltenen Polycarbonat-Formteile sind in bekannter Weise (siehe wieder EP 0 359 953, Seite 12, Zeilen 31 ff und DE-OS 38 33 953, Seite 13, Zeilen 67-68 und Seite 14, Zeilen 1 und 2) technisch brauchbar beziehungsweise verwendbar, beispielsweise im Automobilbereich und im Elektrobereich.

## Beispiele

### Beispiel 1

Herstellung eines Bischlorkohlensäureestergemisches der Formel (Va) analog Macromolecules 1991, 24, Seiten 3035 - 3044.

In eine Mischung aus 310 g (1 Mol) Bisphenol (II), 80 ml $H_2O$ und 1,2 Liter $CH_2Cl_2$ werden bei 0°C 300 g Phosgen in 150 Minuten eingeleitet. Gleichzeitig wird 25 gew.-%tige Natronlauge so zudosiert, daß der pH-Wert der Mischung zwischen 2 und 5 liegt. Man braucht dafür etwa 1 Liter Natronlauge. Nach der Phosgeneinleitung wird der pH-Wert auf 8-9 eingestellt und solange Stickstoff eingeleitet, bis die Lösung phosgenfrei ist. Nach der Phasentrennung wird die organische Phase mit 1 N HCl und dann mit Wasser gewaschen, über Natriumsulfat gewaschen und eingedampft. Die Ausbeute betrug 327 g. Der Gehalt an hydrolysierbarem Chlor betrug 13,4 % gegenüber dem Wert von 16,3 % des reinen Bischlorkohlensäureesters des Bisphenols (II). Daraus ergibt sich ein Wert für "p" von 1,28.

### Beispiel 2

Herstellung eines cyclischen Oligocarbonatgemisches der Formel (VIa) analog Indian Journal of Technology, 31 (1993) Seiten 234-246.

In einem 1 Liter Kolben werden 200 ml $CH_2Cl_2$, 7 ml $H_2O$, 3 ml 9,75 molare NaOH in $H_2O$ und 2,4 ml Triethylamin vorgelegt und bei 40°C unter kräftigem Rühren synchron 200 ml einer Lösung von 87 g des Bischlorkohlensäureestergemisches des Beispiels 1 in $CH_2Cl_2$, 59 ml der 0,75 molaren NaOH in $H_2O$ und 25 ml einer 10 gew.-%igen Lösung von Triethylamin in Methylenchlorid innerhalb 28 Minuten zudosiert, wobei die Dosierung der Bischlorkohlensäureesterlösung durch eine Peristaltikpumpe unterhalb des Flüssigkeitsspiegels erfolgt. Nach Phasentrennung wird mit 1 N HCl und dreimal Wasser gewaschen und die organische Phase eingedampft. Man erhält in nahezu quantitativer Ausbeute ein Material, das zu mindestens 85 % aus Cyclen besteht. (Bestimmung durch HPLC (high performance liquid chromatography). Eine weitere Reinigung kann durch Umfällen aus Aceton erfolgen.

$\overline{M}_n$ (Zahlenmittelmolekulargewicht) 1440, $\overline{M}_w$ (Gewichtsmittelmolekulargewicht) 2350. Beides ermittelt durch Gelpermeationschromatographie.

Die Glastemperatur beträgt 211°C-213°C (amorphes Gemisch), gemessen durch differentiale Thermoanalyse.

Ein Mol der erhaltenen Oligocarbonatgemische enthält 0,03 Mol Endgruppen.

Die phenolischen OH-Endgruppen werden photometrisch nach Anfärbung mit $TiCl_4$, und die Stickstoff-haltigen Endgruppen durch Verbrennung/Chemilumineszenz bestimmt.

**Beispiel 3**

Ringöffnende Polymerisation

Man erhitzt unter Stickstoff 10 g des Oligocarbonatgemisches des Beispiels 2 mit 100 ppm Tetrabutylammonium-tetraphenylboranat für 15 Min. auf 340°C.

Man erhält ein Polycarbonat der relativen Lösungsviskosität von 1,35 (0,5 % in Methylenchlorid bei 20°C) und einer Glastemperatur von 238°C.

**Beispiel 4** (Vergleich)

Blends aus linearem Homopolycarbonat des 2,2-Bis-(4-hydroxyphenyl)-propans (relative Lösungsviskosität 1.26, gemessen in $CH_2Cl_2$ bei 20°C und einer Konzentration von 0,5 g/100 ml, $\overline{M}_w$ = 27 100, $\overline{M}_n$ = 13 500, gemessen durch Gelpermeationschromatographie, Glastemperatur 147,3°C) und Oligocarbonatgemisch des Beispiels 2 in verschiedenen Gewichtsanteilen (0 %, 10 %, 20 %, ... 100 %) wurden in Methylenchlorid als Lösungsmittel hergestellt.

Filme aus den Lösungsblends wurden bei 120°C getrocknet, die Glastemperaturen wurden durch differentiale Thermoanalyse bestimmt und in Abbildung 1 gegen Gewichtsanteile des Oligocarbonatgemisches aufgetragen.

**Beispiel 5**

Blends aus linearem Homopolycarbonat des 2,2-Bis-(4-hydroxyphenyl)-propans (Spezifikationen wie oben in Beispiel 4) und Oligocarbonatgemisch des Beispiels 2 in verschiedenen Gewichtsanteilen (0 %, 10 %, 20 %, ... 100 %) wurden in einem Haakekneter in Gegenwart von 150 ppm Dibutylzinnoxid bei 300°C und 200 Umdrehungen/Min., 10 Min. unter Stickstoff hergestellt. Das gebildete Produkt wurde nach Kühlung pulverisiert. Die Glastemperaturen wurden durch differentiale Thermoanalyse gemessen und in Abbildung 1 gegen Gewichtsanteile des Oligocarbonatgemisches aufgetragen.

Abbildung 1 zeigt, daß die Gemische nach Reaktion (Beispiel 5) höhere Glastemperaturen zeigen als die physikalischen Blends in Beispiel 4 mit gleichen ursprünglichen Zusammensetzungen.

**Patentansprüche**

1. Verwendung von Bischlorkohlensäureestergemische der Formel (V)

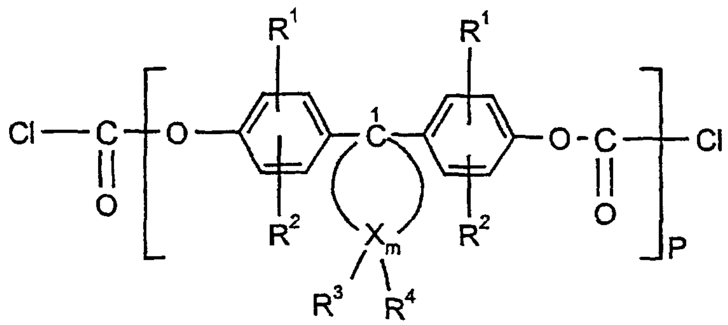

(V),

worin

| | |
|---|---|
| $R^1$ und $R^2$ | unabhängig voneinander Wasserstoff, Halogen, $C_1$-$C_8$-Alkyl, $C_5$-$C_6$-Cycloalklyl, $C_6$-$C_{10}$-Aryl, und $C_7$-$C_{12}$-Aralkyl, |
| m | eine ganze Zahl von 4 bis 7, |
| $R^3$ und $R^4$ | für jedes X individuell, unabhängig wählbar voneinander Wasserstoff oder $C_1$-$C_6$-Alkyl und |
| x | Kohlenstoff bedeuten, |

mit der Maßgabe, dass an mindestens einem Atom X, $R^3$ und $R^4$ gleichzeitig Alkyl bedeuten, und

worin "p", im Mittel eine Zahl von 1 bis 4 ist,

zur Herstellung von Gemischen von cyclischen, aromatischen Oligocarbonaten.

**2.** Verfahren zur Herstellung von Gemischen cyclischer Oligocarbonate der Formel (VI)

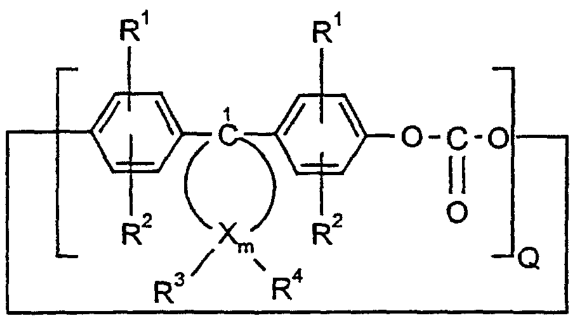

(VI),

worin $R^1$ bis $R^4$, "m" und "X" die für Formel (V) in Anspruch 1 genannte Bedeutung haben und "q" im Mittel eine Zahl von 2 bsi 20 ist, **dadurch gekennzeichnet, dass** man die Lösung der Bichlorkohlensäureestergemische der Formel (V) des Anspruchs 1 in einem organischen Lösungsmittel und ein organisches Amin synchron zu einem zweiphasigen Gemisch aus einem organischen Lösungsmittel und Wasser bei Temperaturen von 0°C bis 40°C zutropft, wobei gleichzeitig die Lösung oder Suspension von einem Alakalimetall- oder Erdalkalimetall-hydroxid so zugegeben wird, daß der pH-Wert zwischen 7 und 13 liegt.

**3.** Gemische cyclischer Oligocarbonate der Formel (VI) erhältlich nach Anspruch 2.

**4.** Verwendung der Gemische der cyclischen Oligocarbonate der Formel (VI) des Anspruches 3 zur Herstellung von hochmolekularen Polycarbonaten mit $\overline{M}_w$ von mindestens 10 000.

**5.** Verfahren zur Herstellung von hochmolekularen, thermoplastichen aromatischen Polycarbonaten mit $\overline{M}_w$ von mindestens 10 000, die an bifunktionellen Struktuteinheiten nur solche der Formel (VII)

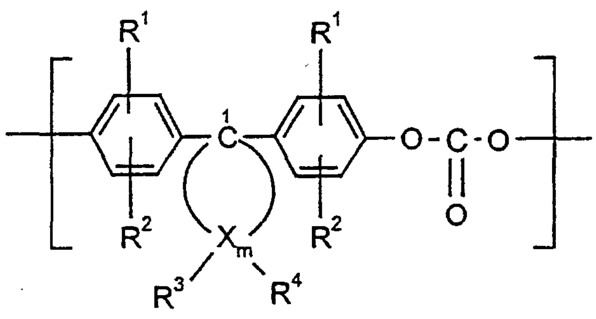

(VII),

haben, worin $R^1$ bis $R^4$, "m" und "X" die für Formel (V) in Anspruch 1 genannte Bedeutung haben, **dadurch gekennzeichnet, daß** man die cyclischen Oligocarbonatgemische der Formel (VI) des Anspruchs 3 in Gegenwart von 0,001 Gew.-% bis 0,1 Gew.-%, bezogen auf Oligocarbonatgemisch der Formel (VI), eines Katalysators 15 Sekunden bis 60 Minuten auf 200°C bis 400°C erhitzt.

**6.** Verwendung der Gemische der cyclischen Oligocarbonate der Formel (VI) des Anspruchs 3 zur Modifizierung von linearen, aromatischen Polycarbonaten aus anderen Diphenolen der Formel (VIII)

$$HO\text{-}Z\text{-}OH \qquad (VIII)$$

worin -Z- ein zweiwertiger, aromatischer Rest mit 6 bis 30 C-Atomen ist, der gegebenenfalls noch -O-, -S- oder

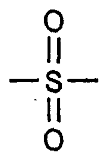

Einheiten und/oder Cl- oder Br-Substituenten enthalten kann, mit $\overline{M}_w$ von 8 000 bis 100 000 und Einfriertemperaturen von 140°C bis 180°C.

**7.** Verfahren zur Herstellung von Gemischen von linearen Polycarbonaten aus solchen mit Struktureinheiten (VII) von Anspruch und solchen mit Struktureinheiten (IX) und linearen Copolycarbonaten mit Struktureinheiten (VII) und (IX), **dadurch gekennzeichnet, daß** man

A) lineare, aromatische Polycarbonate

a) mit Struktureinheiten der Formel (IX)

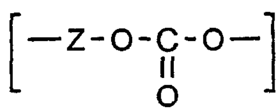 (IX),

worin -Z- ein zweiwertiger, aromatischer Rest mit 6 bis 30 C-Atomen ist, der gegebenenfalls noch -O-, -S- oder

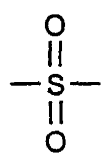

Einheiten und/oder Cl- oder Br-Substituenten enthalten kann,

b) mit Gewichtsmittelmolekulargewichten $\overline{M}_w$ von 8 000 bis 100 000, und

c) mit Einfriertemperaturen von 140°C bis 180°C, und

B) Gemische cyclischer Oligocarbonate der Formel (VI) des Anspruchs 3

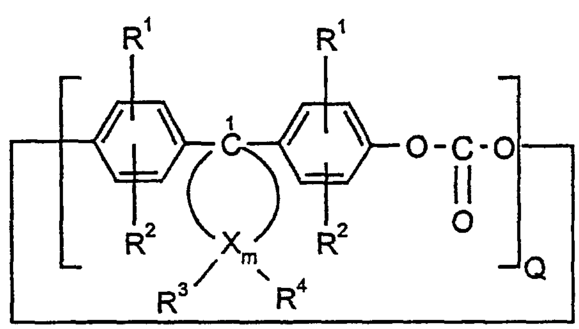 (VI),

worin $R^1$ bis $R^4$, "m" und "X" die für Formel (V) in Anspruch 1 genannte Bedeutung haben und "q" in Mittel

eine Zahl von 2 bis 20 ist, in Gew.-%-Verhältnissen zwischen 0,1 Gew.-% Komponente B) zu 99,9 Gew.-% Komponente A) und 20 Gew.% Komponente B) zu 80 Gew.-% Komponente A), bei Temperaturen von 200°C bis 400°C,

gegebenenfalls in Gegenwart von Katalysatoren, zwischen 10 Sekunden und 60 Minuten in der Schmelze vermischt.

## Claims

1. The use of mixtures of bischlorocarbonic acid esters of formula (V)

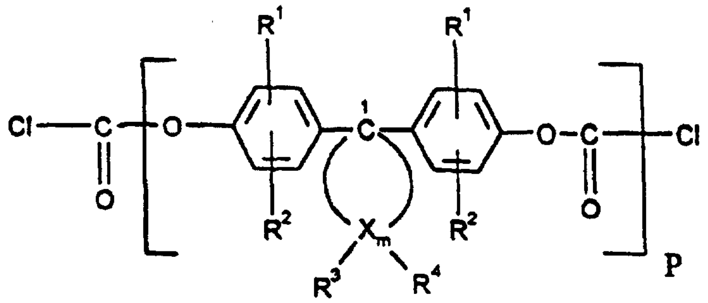

(V),

wherein

R$^1$ and R$^2$, independently of each other, represent hydrogen, a halogen, a $C_1$-$C_8$ alkyl, a $C_5$-$C_6$ cycloalkyl, a $C_6$-$C_{10}$ aryl, and a $C_7$-$C_{12}$ aralkyl,

m is an integer from 4 to 7,

R$^3$ and R$^4$ represent hydrogen or a $C_1$-$C_6$ alkyl, and can be selected individually and independently of each other for each X, and

x represents carbon,

with the proviso that R$^3$ and R$^4$ simultaneously represent alkyl on at least one X atom, and

wherein "p" is a number from 1 to 4 on average,

for the production of mixtures of cyclic aromatic oligocarbonates.

2. A process for producing mixtures of cyclic oligocarbonates of formula (VI)

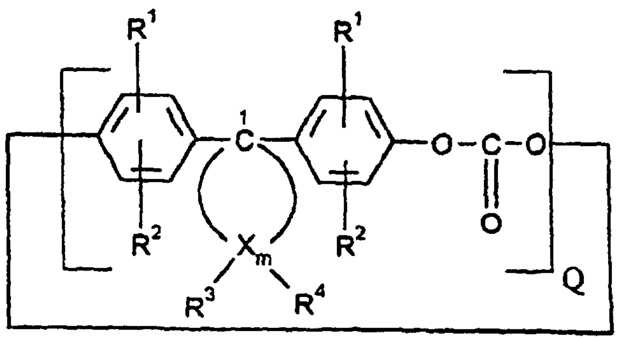

(VI),

wherein R$^1$ to R$^4$, "m" and "X" have the meanings given for formula (V) in claim 1, and "q" is a number from 2 to

20 on average, **characterised in that** a solution of the mixtures of bischlorocarbonic acid esters of formula (V) of claim 1 in an organic solvent, and an organic amine, are added synchronously and drop-wise to a two-phase mixture of an organic solvent and water at temperatures of 0°C to 40°C whilst a solution or suspension of an alkali metal or alkaline earth metal hydroxide is simultaneously added so that the pH is between 7 and 13.

**3.** Mixtures of cyclic oligocarbonates of formula (VI) obtainable according to claim 2.

**4.** The use of the mixtures of cyclic oligocarbonates of formula (VI) of claim 3 for the production of high molecular weight polycarbonates with an $\overline{M}_w$ of at least 10,000.

**5.** A process for producing high molecular weight, thermoplastic, aromatic polycarbonates with an $\overline{M}_w$ of at least 10,000, which contain, as bifunctional structural units, only those of formula (VII)

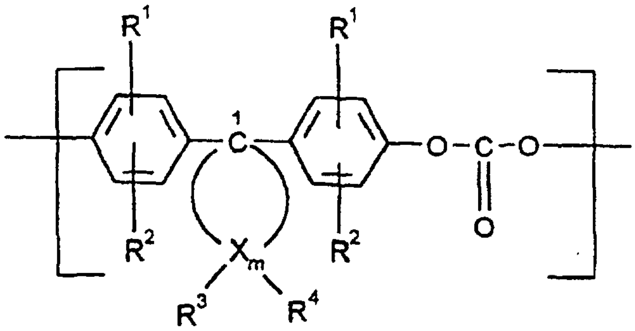

(VII),

wherein $R^1$ to $R^4$, "m" and "X" have the meanings given for formula (V) in claim 1, **characterised in that** the cyclic oligocarbonate mixtures of formula (VI) of claim 3 are heated for 15 seconds to 60 minutes at 200°C to 400°C, in the presence of 0.001 % by weight to 0.1 % by weight, with respect to the oligocarbonate mixture of formula (VI), of a catalyst.

**6.** The use of the mixtures of cyclic oligocarbonates of formula (VI) of claim 3 for the modification of linear aromatic polycarbonates of other diphenols of formula (VIII)

$$HO - Z - OH \qquad (VIII)$$

where -Z- is a divalent aromatic radical which contains 6 to 30 C atoms and which may optionally also contain -O-, -S- or

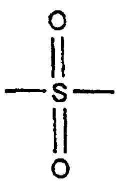

units and/or Cl or Br substituents, with an $\overline{M}_w$ of 8000 to 100,000 and glass transition temperatures of 140°C to 180°C.

**7.** A process for producing mixtures of linear polycarbonates from those which contain structural units (VII) of claim 5 and those which contain structural units (IX), and linear copolycarbonates which contain structural units (VII) and (IX), **characterised in that**

A) linear aromatic polycarbonates

a) which contain structural units of formula (IX)

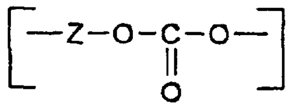

(IX),

wherein -Z- is a divalent aromatic radical which contains 6 to 30 C atoms and which may optionally also contain -O-, -S- or

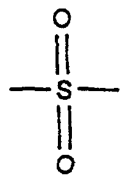

units and/or Cl or Br substituents,

b) which have weight average molecular weights $\overline{M}_w$ of 8000 to 100,000, and

c) which have glass transition temperatures of 140°C to 180°C, and

B) mixtures of cyclic oligocarbonates of formula (VI) of claim 3

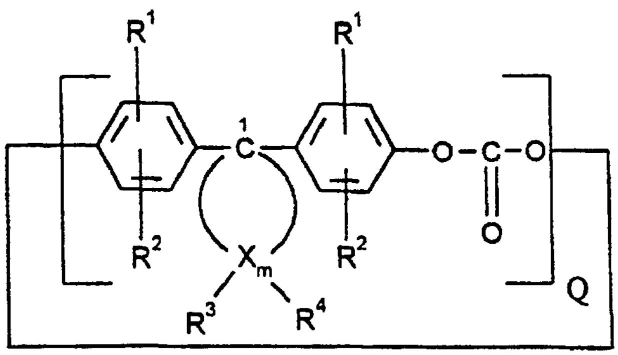

(VI),

wherein $R^1$ to $R^4$, "m" and "X" have the meanings given for formula (V) in claim 1, and "q" is a number from 2 to 20 on average,

are mixed in the melt at temperatures of 200°C to 400°C, in ratios by weight between 0.1 % by weight of component B) to 99.9 % by weight of component A) and 20 % by weight of component B) to 80 % by weight of component A),

optionally in the presence of catalysts, for between 10 seconds and 60 minutes.

## Revendications

**1.** Utilisation des mélanges de bisesters d'acide chlorocarbonique de formule (V) :

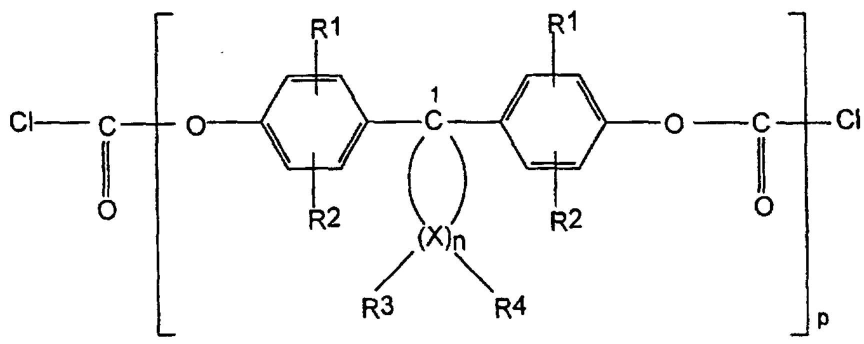

(V)

où

$R^1$ et $R^2$ représentent indépendamment l'un de l'autre, l'atome d'hydrogène, un atome d'halogène, un radical alcoyle en $C_1$-$C_8$, cycloalcoyle en $C_5$-$C_6$, aryle en $C_6$-$C_{10}$, et aralcoyle en $C_7$-$C_{12}$ ;
m représente un nombre entier allant de 4 à 7 ;
$R^3$ et $R^4$ représentent, pour chaque X individuellement, indépendamment l'un de l'autre, l'atome d'hydrogène ou un radical alcoyle en $C_1$-$C_6$, et
X représente un atome de carbone,
avec la condition que sur au moins un atome X, $R^3$ et $R^4$ représentent simultanément un radical alcoyle, et où « p » est en moyenne, un nombre allant de 1 à 4,
pour préparer des mélanges d'oligocarbonates aromatiques, cycliques.

2. Procédé de préparation de mélanges d'oligocarbonates cycliques de formule (VI) :

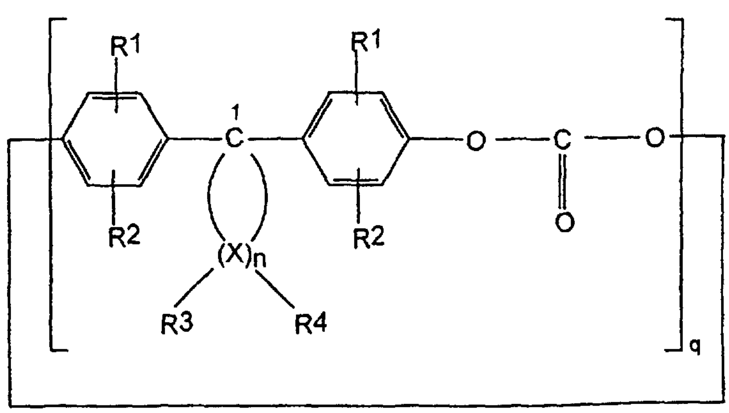

(VI)

où $R^1$ à $R^4$, « m » et « X » ont la signification donnée pour la formule (V) à la revendication 1 et « q » est en moyenne, un nombre allant de 2 à 20, qui est **caractérisé en ce que** l'on ajoute goutte à goutte, la solution du mélange de bisesters d'acide chlorocarbonique de formule (V) suivant la revendication 1, dans un solvant organique et une amine organique, de manière synchrone, à un mélange biphasique d'un solvant organique et d'eau, à une température située dans l'intervalle allant de 0°C à 40°C, où simultanément, on ajoute la solution ou suspension d'un hydroxyde de métal alcalin ou de métal alcalino-terreux, de sorte que le pH se situe dans la gamme allant de 7 à 13.

3. Mélange d'oligocarbonates cycliques de formule (VI) pouvant être obtenu suivant la revendication 2.

4. Utilisation du mélange d'oligocarbonates cycliques de formule (VI) suivant la revendication 3, pour préparer des polycarbonates de poids moléculaire élevé avec $M_w$ égal à au moins 10 000.

5. Procédé de préparation de polycarbonates aromatiques, thermoplastiques, de poids moléculaire élevé avec $M_w$ égal à au moins 10 000, qui ont comme unités structurales bifonctionnelles, seulement celles de formule (VII) :

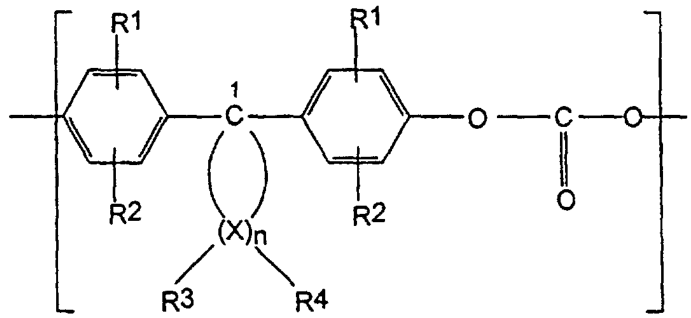

(VII)

où $R^1$ à $R^4$, « m » et « X » ont la signification donnée pour la formule (V) à la revendication 1, qui est **caractérisé en ce que** l'on chauffe le mélange d'oligocarbonates cycliques de formule (VI) suivant la revendication 3, en présence de 0,001% en poids à 0,1% en poids, sur base du mélange d'oligocarbonates de formule (VI), d'un catalyseur, pendant 15 secondes à 60 minutes, à température de 200°C à 400°C.

**6.** Utilisation des mélanges d'oligocarbonates cycliques de formule (VI) suivant la revendication 3, pour modifier des polycarbonates aromatiques, linéaires d'autres diphénols de formule (VIII) :

$$HO - Z - OH \qquad (VIII)$$

où -Z- est un reste aromatiques bivalent ayant 6 à 30 atomes C, qui peut contenir facultativement, des unités -O-, -S- ou $-SO_2-$ et/ou des substituants Cl ou Br, avec $M_w$ allant de 8000 à 100 000 et une température de prise allant de 140°C à 180°C.

**7.** Procédé de préparation de mélanges de polycarbonates linéaires de ceux avec les unités structurales (VII) suivant la revendication 5 et de ceux avec les unités structurales (IX) et de copolycarbonates linéaires avec les unités structurales (VII) et (IX), qui est **caractérisé en ce que** l'on mélange :

A) des polycarbonates aromatiques linéaires

a) avec des unités structurales de formule (IX) :

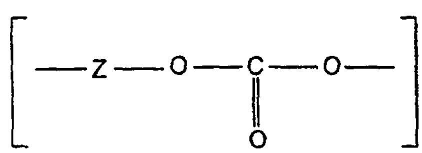

(IX)

où -Z- est un reste aromatique bivalent, avec 6 à 30 atomes C, qui peut contenir facultativement, des unités -O-, -S- ou $-SO_2-$ et/ou des substituants Cl ou Br,
b) avec un poids moléculaire moyen en poids $M_w$ allant de 8000 à 100 000, et
c) avec une température de prise allant de 140°C à 180°C, et

B) des mélanges d'oligocarbonates cycliques de formule (VI) suivant la revendication 3 :

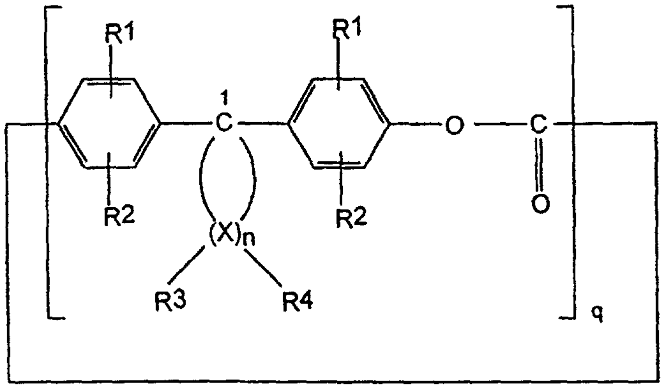

(VI)

où $R^1$ à $R^4$, « m » et « X » ont la signification donnée pour la formule (V) à la revendication 1, et « q » est en moyenne, un nombre allant de 2 à 20,

en des proportions pondérales allant de 0,1% en poids du composant B) pour 99,9% en poids du composant A) à 20% en poids du composant B) pour 80% en poids du composant A), à des températures allant de 200°C à 400°C,

facultativement en présence d'un catalyseur, pendant 10 secondes à 60 minutes, à l'état fondu.

Fig. 1

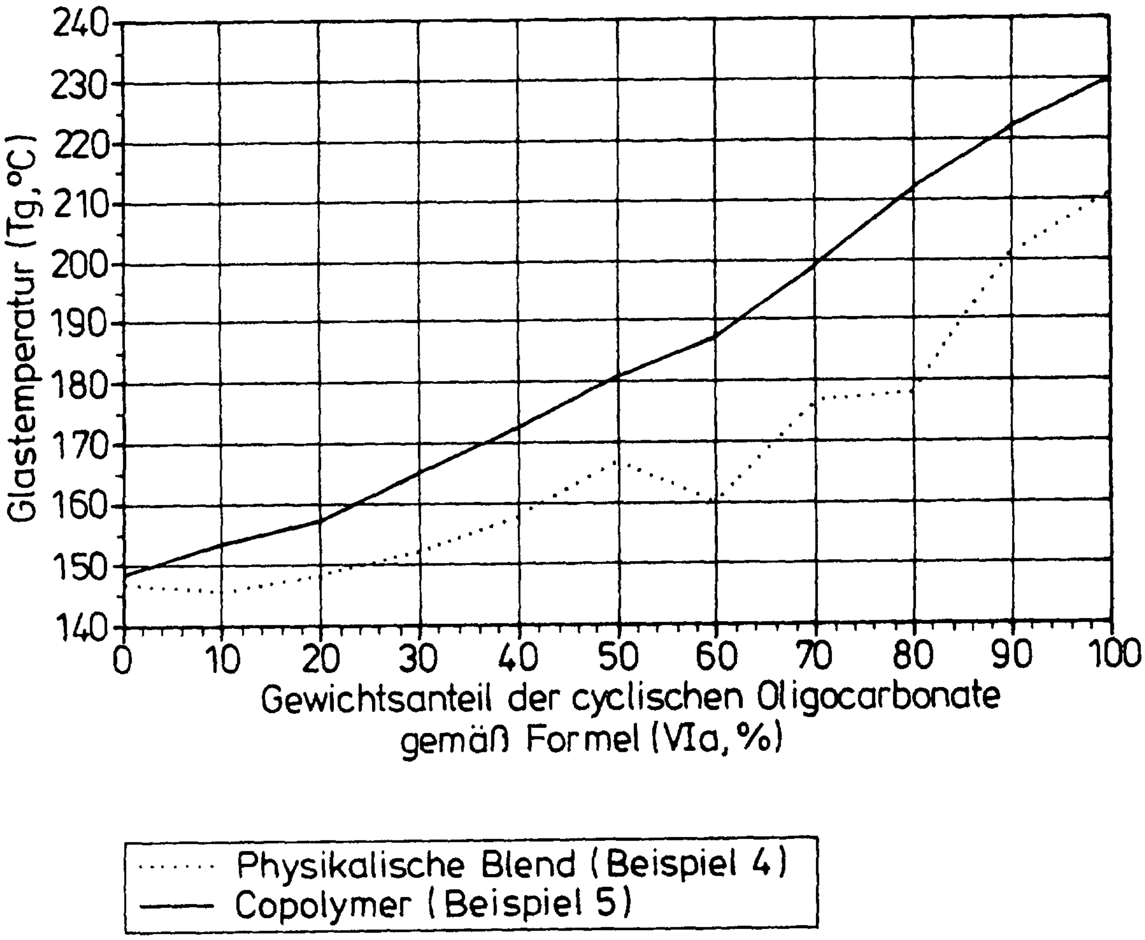
240
230
220
210
200
190
180
170
160
150
140
Glastemperatur (Tg, °C)
0
10
20
30
40
50
60
70
80
90
100
Gewichtsanteil der cyclischen Oligocarbonate gemäß Formel (VIa, %)
Physikalische Blend (Beispiel 4)
Copolymer (Beispiel 5)